(19) Europäisches Patentamt
European Patent Office
Office européen des brevets

(11) Publication number: **0 348 509 B1**

(12) **EUROPEAN PATENT SPECIFICATION**

(45) Date of publication of patent specification: **24.03.93**

(51) Int. Cl.$^5$: **A61K 31/12**, A61K 31/185, A61K 31/195, A61K 31/35, A61K 31/70, A61K 35/78

(21) Application number: **88907791.3**

(22) Date of filing: **12.09.88**

(86) International application number: **PCT/JP88/00919**

(87) International publication number: **WO 89/05141 (15.06.89 89/13)**

(54) **ANTI-RETROVIRAL DRUG.**

(30) Priority: **10.12.87 JP 311038/87**
**21.12.87 JP 321516/87**
**21.12.87 JP 321517/87**
**21.12.87 JP 321518/87**
**05.09.88 JP 220346/88**

(43) Date of publication of application: **03.01.90 Bulletin 90/01**

(45) Publication of the grant of the patent: **24.03.93 Bulletin 93/12**

(84) Designated Contracting States: **AT BE CH DE FR GB IT LI NL**

(56) References cited:
**FR-A- 2 471 783**
**JP-A- 5 830 642**

**Chemical Abstracts, vol. 90, no. 9, Abstract no. 67141y, 26 Feb 1979, Columbus, Ohio, US ; I.Musci et al., Orvostud. Aktual. Probl., 31, 51(1978)**

(73) Proprietor: **TSUMURA & CO.**
**4-10, Nihonbashi 3-chome**
**Chuo-ku, Tokyo 103(JP)**

(72) Inventor: **ONO, Katsuhiko Hikarigaoka Haitsu A-110**
**1-135, Heiwagaoka Meito-ku Nagoya-shi**
**Aichi 465(JP)**
Inventor: **FUKUSHIMA, Masanori 1-5-9, Jiyugaoka, Chikusa-ku**
**Nagoya-shi**
**Aichi 464(JP)**
Inventor: **NAKANE, Hideo Aichi-ken Gan Center Hoankosha A**
**1-1, Kanokoden Chikusa-ku Nagoya-shi**
**Aichi 464(JP)**
Inventor: **NAKAGAMI, Shizuo Tsumura Juntendo, Inc.**
**12-7, Niban-cho Chiyoda-ku**
**Tokyo 102(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

Chemical Abstracts, vol. 102, no. 11, Abstract no. 92800m, 18 March 1985, Columbus, Ohio, US; T.N. Kaul et al., J. Med. Virol., 15, 71 (1985)

Yano Michiaki [insho Oyo Kanpo Shoho Kaisetsu], 20 July 1977, Sogen-sha, pp.263-271

Chemical Abstracts, vol. 82, no. 13, Abstract no. 80163m, 31 March 1975, Columbus, Ohio, US; A.Koda, Taisha 10, 730 (1973)

Chemical Abstracts, vol. 96, no.17, Abstract no. 137733t, 26 April 1982, Columbus, Ohio, US, Y. Kimura et al., Chem. Pharm. Bull., 30, 219 (1982)

Chemical Abstracts, vol. 101, no.17, Abstract no. 143768w, 22 Octo ber 1984, Columbus, Ohio, US; M.Kubo et al., Chem. Pharm. Bull., 32, 2724 (1984)

Chemical Abstracts, vol. 103, no. 15, Abstract no. 115972f, 14 October 1985, Columbus, Ohio, UD; M. Kubo et al.

Chemical Abstracts, vol. 92, no. 20, Abstract no. 169246v, 19 May 1980, Columbus, Ohio, US, Madaus, Dr., und Co., DE 2, 826, 642(1980)

Chemical Abstracts, vol. 104, no.23, Abstract no. 199515r, 9 June 1986, Columbus, Ohio, D. Lorenz et al., Methods. Find. Exp. Clin. Pharmacol., 7, 637 (1985)

Chemical Abstracts, vol. 90, no. 8, Abstract no. 61242k, 19 Feb 1979, Columbus, Ohio, US, R.S. Schreiber & US-4105558(1978)

ANN. NEW YORK ACADEMY OF SCIENCE, vol. 284, 1976, pp. 358-364 ; I.BELADI et al.:"Activity of some flavonoids against viruses"

ARCHIVES OF VIROLOGY, vol. 57, 1987, pp. 255-260, Springer Verlag ;A. VECKENSTEDT et al.:"Effects of treatment with certain flavonoids on mengo virus-induced encephalitis in mice"

EXPERIENTIA, vol. 41, no.7, 1985, pp.930-931, Birkhäuser Verlag, Basel, CH ; I. MUSCI et al.:"Inhibition of virus multiplication and alteration of cyclic AMP level in cell cultures by flavonoids"

CHEM. PHARM. BULL, vol. 33, no.9, 1985, pp. 3881-3885 ; Y. TSUCHIYA et al.:"Antiviral activity of natural occuring flanoids in vitro"

DEUTSCHE APOTHEKER ZEITUNG, vol. 126, no. 35, August 26, 1986, pp. 1811-1816 ; O.SCHIMMER et al.:"Flanoide, Ihre Rolle als biologisch aktive Naturstoffe"

EXPERIENTIA, vol. 36, no.3, 1980, page 304, Birkhäuser Verlag, Basel, CH ; R. POMPEI et al. :"Antiviral activity of glycyrrhizic acid"

JPN. J. CANCER RES., vol. 78, no.8, August 1987, pp. 767-771 ; H. NAKASHIMA et al. :"A new antihuman immunodeficiency virus substance, glycyrrhizin sulfate ; endowment of glycyrrhizin with reverse transcriptase-inhibitory activity by chemical modification"

ANTIVIRAL RESEARCH, vol. 7, 1987, pp. 35-42, Elsevier Science Publishers B.V. ; R. VRIJSEN et al.:"The poliovirus-induced shut-off of cellular protein synthesis persists in the presence of 3-methylquercetin, a flavonoid which blocks viral protein and RNA synthesis"

JPN. J. ALLERGY, vol. 35, no. 11, 1986, pp. 1119-1121 ; Y. MIZOGUCHI et al.:"The effects of Xiao-CHAI-Hu-Tang (Syosaiko-To) on lymphokine activated killer (lak) cell activity"

ANTIVIRAL RES., vol. 12, no. 2, 1989, pp. 99-110, Elsevier Science Publishers B.V.(Biomedical Division), G. SPEDDING et al.:"Inhibition of reverse transcriptases by flavonoids"

ANTIVIRAL RES., vol 7, 1987, pp. 127-137, Elsevier Science Publishers B.V. ; M. ITO et al.:"Inhibitory effect of glycyrrhizin on the in vitro infectivity and cytopathic activity of the human immunodeficiency virus (HIV(HTLV-III/LAV)

BIOORGANIC CHEMISTRY, vol. 14, no.1, 1986, pp. 55-69, Academic Press, Inc.; M.ROSSI et al.:"The crystal and molecular structure of quercetin : a biologically active and naturally occuring flavonoid"

Chemical Abstracts, vol. 76, no. 5, Abstract no. 21262w, 31 Jan. 1972, Columbus, Ohio, US; A. Koda et al.

Inventor: **HIGUCHI, Hirotaka Tsumura Juntendo, Inc.**
**3586, Yoshiwara Ami-machi Inashiki-gun**
**Ibaragi 300-11(JP)**
Inventor: **MORI, Kazuya Tsumura Juntendo, Inc.**
**3586, Yoshiwara Ami-machi Inashiki-gun**
**Ibaragi 300-11(JP)**
Inventor: **SAKATA, Toshiya Tsumura Juntendo, Inc.**
**3586, Yoshiwara Ami-machi Inashiki-gun**
**Ibaragi 300-11(JP)**

(74) Representative: **Stuart, Ian Alexander et al**
**MEWBURN ELLIS 2 Cursitor Street**
**London EC4A 1BO (GB)**

## Description

The present invention relates to an anti-retroviral agent effective for the remedy of viral diseases caused by human retroviruses.

Vaccination has generally been adopted as a means of preventing infection with a virus, and with the development of the scientific techniques, agents for controlling the propagation of certain viruses have been developed.

Among various viruses, HIV (human immunodeficiency virus) causing an acquired immune deficiency syndrome (AIDS) and HTLV-1 (human T-cell leukemia virus type 1) causing human T-cell leukemia are known as retroviruses.

A retrovirus is a virus containing an RNA-dependent DNA synthesis enzyme (hereinafter referred to as "reverse transcriptase") in virus particles, and this virus grows in the following manner.

(1) After the virus infects a host cell, viral RNA is transcribed to viral DNA by the action of reverse transcriptase.

(2) Viral DNA is integrated in the chromosomal DNA of the host cell, and viral mRNA is then synthesized by a cellular RNA polymerase of the host cell.

(3) Viral proteins are produced by viral mRNA.

(4) The proteins formed by the above-mentioned mRNA bond to genome RNA to form a filial virus, and this filial virus leaves the cell.

A medicinal agent having an epoch-making curative effect to human diseases caused by retroviruses has not been developed, and the development of such a medicinal agent is urgently required.

Archives of Virology, 57,(1978) pp 255-260 and Chem. Abstracts 102,(1985)pp 320, 102:92800m disclose that some flavonoids including quercetin and hesperidin have anti-viral effects against certain viruses (none of which are retroviruses).

Antiviral Research, 7,(1987) pp 127-137 discloses an action of 3-methylquercetin on poliovirus.

Experientia, 36,(1980) pp 304 discloses that glycyrrhizic acid inhibits the growth of several DNA and RNA viruses in cell cultures and inactivates Herpes simplex 1 virus irreversibly.

A primary object of the present invention is to provide an anti-retroviral agent effective for the remedy of human diseases caused by retroviruses.

The present inventors investigated the anti-retroviral effect of various herbal drugs and plant components, and as a result, found that Xiao-Chai-Hu-Tang, which is a Chinese medicine preparation comprising Bupleuri radix, Scutellariae radix, Glycyrrhizae radix, Ginseng radix, Zingiberis rhizoma, Zizyphi fructus and Pinelliae tuber, baicalein and baicalin extracted and isolated from a herb drug Scutellariae radix, quercetin extracted and isolated from a herb drug Sophora japonica, hesperetin extracted and isolated from a herb drug Aurantii nobilis pericarpium, and alizarin (1,2-dihydroxyanthraquinone), which is a red pigment of madder, a plant belonging to the family Rubia, and a derivative thereof, have an anti-retroviral effect, and the present invention was completed based on this finding.

More specifically, in accordance with the present invention, there is provided the use in manufacturing an anti-retroviral agent of, as an effective ingredient, at least one member selected from Xiao-Chai-Hu-Tang, baicalein, baicalin, quercetin, hesperetin, and alizarin and derivatives and pharmacologically acceptable salts thereof, represented by the following formula I (collectively referred to as "compound of formula I" hereinafter):

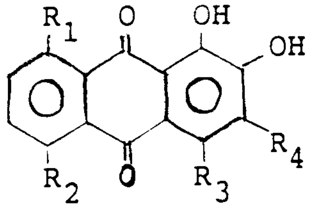

I

wherein $R_1$, $R_2$, and $R_3$, which may be the same or different, stand for a hydrogen atom or a hydroxyl group, and $R_4$ stands for a hydrogen atom, a sulfonic acid group or a dicarboxymethylaminomethyl group.

The constituent herb drugs, doses and extraction methods of Xiao-Chai-Hu-Tang are described in classical literature references concerning Chinese medicine preparations (such as Shang-Han-Lun and Jin-Kui-Yao-Lue), and this preparation has been used for the remedy of liver troubles, chronic gastroenteric

troubles, postpartal recovery deficiency, and other diseases, but it has not been known that Xiao-Chai-Hu-Tang has an anti-retroviral effect.

Any of the preparations of Xiao-Chai-Hu-Tang formed according to recipes of herb drugs disclosed in classic literature references such as Shang-Han-Lun and Jin-Kui-Yao-Lue can be used as Xiao-Chai-Hu-Tang in the present invention.

With regard to the mixing ratios of the respective herb drugs, Xiao-Chai-Hu-Tang preferably comprises 4 to 7 parts by weight of Bupleuri radix, 3 parts by weight of Scutellariae radix, 2 parts by weight of Glycyrrhizae radix, 2 to 3 parts by weight of Ginseng radix, 1 part by weight of Zingiberis rhizoma, 2 to 3 parts by weight of Zizyphi fructus, and 4 to 5 parts by weight of Pinelliae tuber.

Xiao-Chai-Hu-Tang, which is formed, for example, by boiling 7 g of Bupleuri radix, 3 g of Scutellariae radix, 2 g of Glycyrrhizae radix, 3 g of Ginseng radix, 1 g Zingiberis rhizoma, 3 g of Zizyphi fructus and 5 g of Pinelliae tuber in 600 ml of water until the volume is reduced to 350 ml, removing the dregs, and making a decoction of only the medicinal liquid until the volume is reduced to 200 ml, can be divided into three dosages, to be taken three times per day, but a dry extract powder or a Chinese herb extract medicine formed from the above liquid extract can be used as the anti-retroviral agent in view of ease of administration and portability thereof.

In view of the pharmacological effect desired preferably the Xiao-Chai-Hu-Tang prepared by the following method is used.

More specifically, according to the teachings of Shang-Han-Lun and Jin-Kui-Yao-Lue, 7 g of Bupleuri radix, 3 g of Scutellariae radix, 2 g of Glycyrrhizae radix, 3 g of Ginseng radix, 1 g of Zinziberis rhizoma, 3 g of Zizyphi fructus, and 5 g of Pinelliae tuber are mixed with distilled water in an amount of 10 to 12 times the amount of the herb drug mixture, extraction is carried out at 95 to 100°C for about 60 minutes, solid-liquid separation is carried out, and the obtained liquid is spray-dried to obtain a dry extract powder of Xiao-Chai-Hu-Tang (4.5 g of the dry extract powder contains 25.0 to 52.0 mg of glycyrrhizin, 90 to 210 mg of baicalin, and 2.3 to 6.9 mg of saikosaponin b2).

For the formulation, an excipient, an adjuvant, and other additives customarily used in the pharmaceutical field are added to the dry extract powder, and the mixture is formed into a powder, a granule, a tablet or a capsule, by methods customarily used in the pharmaceutical field.

A specific example of an anti-retroviral agent comprising Xiao-Chai-Hu-Tang as the effective ingredient will now be described.

## Specific Example

To 7 g of Bupleuri radix, 3 g of Scutellariae radix, 2 g of Glycyrrhizae radix, 3 g of Ginseng radix, 1 g of Zingiberis rhizoma, 3 g of Zizyphi fructus, and 5 g of Pinelliae tuber was added 300 ml of distilled water, and extraction was carried out at 100°C for 60 minutes. A solid-liquid separation was effected by centrifugal separation, and the supernatant was spray-dried below 50°C to obtain a dry extract powder of Xian-Chai-Hu-Tang. When the amounts of the components contained in 4.5 g of the dry extract powder were determined, it was found that 42.5 mg of glycyrrhizin, 160 mg of baicalin, and 4.5 mg of saikosaponin b2 were contained.

It is known that baicalein and baicalin have an anti-inflammatory action, an anti-allergic action, and an anti-hypertensive action, but it has not been known that these compounds have an anti-viral action, especially an anti-retro-viral action.

It is also known that quercetin and hesperetin have an anti-inflammatory action, an anti-allergic action, and an anti-hypertensive action, and that these compounds control the growth of a DNA virus, but it has not been known that these compounds have an anti-retroviral effect on a retrovirus which has a reverse transcriptase and grows through this reverse transcriptase.

Baicalin and baicalein are compounds represented by the following chemical structural formula II and are marketed by Wako Junyaku Kabushiki Kaisha:

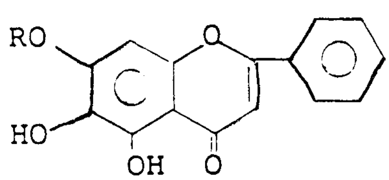

II

wherein if R stands for H, the compound is baicalein and if R stands for $C_6H_9O_6$ (glucuronic acid residue), the compound is baicalin.

Quercetin is a compound represented by the following chemical structural formula III and is marketed by Tokyo Kasei Kogyo Kabushiki Kaisha:

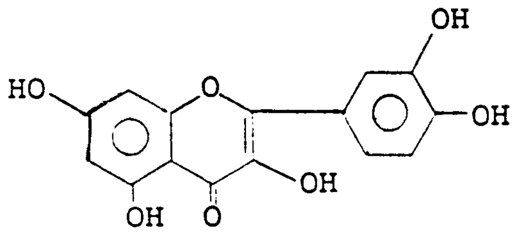

III

Hesperetin is a compound represented by the following chemical structural formula IV and is marketed by Tokyo Kasei Kogyo Kabushiki Kaisha:

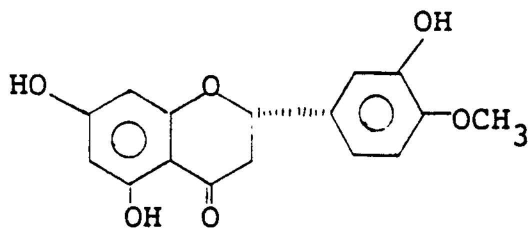

IV

The compound of formula I used in the present invention is known as a pigment, but it has not been known that the compound has an anti-viral effect, especially an anti-retroviral effect.

As the compound of formula I, alizarin that can be easily extracted and isolated from madder, a plant belonging to the family rubia, can be directly used. Derivatives of alizarin can be easily prepared from this alizarin, and derivatives marketed as reagents also can be used as the compound of formula I.

Specific examples of the compound of formula I are shown below.

| | $R_1$ | $R_2$ | $R_3$ | $R_4$ |
|---|---|---|---|---|
| 1 | H | H | H | H |
| 2 | H | H | H | $CH_2N(CH_2COOH)_2$ |
| 3 | H | H | H | $SO_3Na$ |
| 4 | OH | OH | H | H |
| 5 | H | H | OH | H |

(1) alizarin (1,2-dihydroxyanthraquinone)
(2) alizarin complexone (1,2,-dihydroxy-3-dicarboxymethylamino-methylanthraquinone)
(3) Alizarine Red S (1,2-dihydroxy-3-sulfonyl-anthraquinone)
(4) quinalizarin

(1,2,5,8-tetrahydroxyanthraquinone)
(5) purpurin (1,2,4-trihydroxyanthraquinone)

With reference to the following experiments, the anti-retroviral effect of the anti-retroviral agent of the present invention will now be explained.

Experiment 1

Cells infected with mouse leukemia virus were cultured, and the reverse transcriptase was separated and purified by the method of Nakajima et al. [COMPARATIVE LEUKEMIA RESEARCH 1973, LEUKEMOGENESIS, ED. Y. ITO AND R.M. DUTCHER, UNIV. OF TOKYO PRESS TOKYO/KARGER, BASEL, pp. 603-605 (1975)], and then a reaction mixture liquid having the following composition was prepared.

| | |
|---|---|
| Reverse transcriptase | 1 unit/ml |
| Polyadenylic acid / oligothymidylic acid duplex [polyadenylic acid (supplied by Pharmacia / oligothymidylic acid (supplied by Pharmacia) = base ratio 4/1] as temperate / primer | 2 μg/ml |
| Tris-hydrochloric acid (pH 8.0) | 50 mM |
| Dithiothreitol | 5 mM |
| Potassium chloride | 50 mM |
| Manganese chloride | 0.2 mM |
| [$^3$H]-Deoxythymidine triphosphate (referred to as "[$^3$H]dTTP hereinafter] | 0.01 mM (400 cpm/pmol) |
| Glycerol | 15 (v/v)% |
| Distilled water | appropriate amount |

Note

By "1 unit" is meant the unit of the relative activity of the reverse transcriptase required to consume 1 nmol of dNTP (deoxynucleic acid phosphate at 37°C for 1 hour.

Then the dry extract powder obtained in the above-mentioned Specific Example, baicalein, baicalin, quercetin or hesperetin and refined water were added to 20 ml of the above-mentioned reaction mixture liquid so that the total volume was 50 μl, the incorporation of the radioactivity of [$^3$H]dTTP into the acid-insoluble fraction was measured by a Beckmann scintillation counter, and the percent inhibition was calculated as the reverse transcriptase activity with respect to each concentration. The results are shown in Tables 1 through 4.

Table 1

| Dry extract powder (μg/ml) obtained in Specific Example | Percent inhibition (%) |
|---|---|
| 5 | 0.53 |
| 10 | 12.02 |
| 20 | 19.25 |
| 50 | 37.32 |
| 100 | 84.51 |
| 200 | 93.5 |
| 500 | 99.5 |

Table 2

| Concentration (μg/ml) | Percent inhibition (%) of baicalein | Percent inhibition (%) of baicalin |
|---|---|---|
| 0.1 | 29 | - |
| 0.2 | 35 | - |
| 0.5 | 74 | - |
| 1 | 92 | 19 |
| 2 | 98 | 27 |
| 5 | 100 | 73 |
| 10 | 100 | 90 |
| 20 | 100 | 100 |

Table 3

| Concentration (µg/ml) | Percent inhibition (%) of Quercetin |
|---|---|
| 1 | 77 |
| 2 | 96 |
| 5 | 99 |
| 10 | 100 |
| 20 | 100 |
| 50 | 100 |

Table 4

| Concentration (µg/ml) | Percent inhibition (%) of hesperetin |
|---|---|
| 1 | 99 |
| 2 | 100 |
| 5 | 100 |
| 10 | 100 |
| 20 | 100 |
| 50 | 100 |

Experiment 2

MT-2 cells were cultured at a concentration of 3 x $10^4$ cells/ml in a culture medium of RPMI 1640 (supplied by Gibco Co.) containing 10% FCS (supplied by Gibco Co.). After 2 days, the MT-2 cells were infected with HIV at a ratio of 200 cells per HIV, were distributed into groups to which 5 µg/ml of baicalein, quercetin or hesperetin was added and non-added groups (control groups), and the effect of the addition was determined. After 1 week, the number of living cells was counted, and it was confirmed that the modification of MT-2 cells with HIV was inhibited at a ratio of at least 90%.

Experiment 3

A reaction mixture liquid having the following composition was prepared.

| | |
|---|---|
| Reverse transcriptase (Rous Associated Virus-2 supplied by Takara Shuzo) | 2 units/ml |
| Polyadenylic acid/oligothymidylic acid duplex [polyadenylic acid (polyrA) (supplied by Pharmacia), oligothymidylic acid $(dT)_{12-18}$ (supplied by Pharmacia)] as temperate/primer | 10 µg/ml |
| Tris-hydrochloric acid (pH 8.3) | 50 mM |
| Potassium chloride | 50 mM |
| Magnesium chloride | 10 mM |
| $[^3H]$dTTP (40 ci/mmol) (1.0 mCi/ml) | 0.2 µM |
| Deoxythymidine triphosphate | 9.8 µM |
| Distilled water | appropriate amount |

A reaction of 50 µl of the reaction mixture liquid was carried out at 37°C for 30 minutes. The reaction product DNA was determined by the ion exchange filter paper method, the incorporation of the radioactivity of $[^3H]$dTTP into DNA was measured by a Beckmann scintillation counter, and the 50% inhibition concentration ($IC_{50}$) of each test substance was calculated as the reverse transcriptase activity. The results are shown in Table 5.

Table 5

| Test Substance | $IC_{50}$ ($\mu$g/ml) |
|---|---|
| alizarin | 12.5 |
| alizarin complexone | 3.8 |
| Alizarine Red S | 7.0 |
| quinalizarin | 7.7 |
| purpurin | 17.5 |

Experiment 4

A 24-well microplate was charged with 3.5 x $10^5$ cells per well of primary chicken embryo fibroblasts, and the fibroblasts were cultured at 37°C in an atmospher of 5% $CO_2$ for 6 hours. Then an RSV. SR-A strain having an infection multiplicity (M.O.I.) of about 2.0 x $10^{-4}$ and the test substance were added, and culturing was continued for further 2 hours. The cells were washed and the test substance and an agar culture medium were added, and then culturing was continued at 37°C in an atmospher of 5% $CO_2$ for further 8 days. The number of foci was counted, and the results are shown as $IC_{50}$ in Table 6.

Table 6

| Test Substance | $IC_{50}$ ($\mu$g/ml) |
|---|---|
| alizarin complexon | 4.3 |
| Alizarine Red S | 2.1 |

From the results of Experiments 1 through 4, it was confirmed that the anti-retroviral agent of the present invention has an anti-retroviral effect based on the action of inhibiting the reverse transcriptase activity.

Namely, the anti-retroviral agent of the present invention controls the growth of the retrovirus by inhibiting the reverse transcriptase activity necessary for the growth of the retrovirus. Accordingly, the agent of the present invention can be applied to any retrovirus.

As specific examples of the retroviruses, there can be mentioned leukemia virus, sarcoma virus, mammary tumor virus, visna virus, maedic virus, HIV and HTLV-1.

The compound of formula I has not only an action of inhibiting the reverse transcriptase activity but also an action of controlling the retrovirus-producing activity of retrovirus-infected cells.

An experiment demonstrating this action of the retrovirus-producing activity will now be described.

Experiment 5

The test substance and 1 x $10^5$ cells per well of MT-2 cells were charged in a 24-well microplate, and culturing was conducted for 4 days. Then the MT-2 cell suspension in each well was subjected to centrifugal separation, and the supernatant was extracted with phenol to obtain RNA of HTLV-1. This RNA and a known amount of HTLV-1 cDNA were plotted on a nitrocellulose paper, a comparative determination was carried out according to the hybridization using $^{32}$P-labelled HTLV-1 cDNA, and the hydridization rate by the test substance was calculated by comparing the amount of the produced virus with that of the control.

The results are shown as the hybridization rate (%) in Table 7.

Table 7

| Test Substance | Amount Added (μg/ml) | Hybridization Rate (%) |
|---|---|---|
| Alizarine Red S | 20 | 61.7 |
| purpurin | 5 | 61.3 |

From the results of Experiment 5, it was confirmed that the compound of formula I has an effect of controlling the retrovirus-producing activity of retrovirus-infected cells.

When the acute toxicity of the anti-retroviral agent of the present invention comprising Xiao-Chai-Hu-Tang by oral administration was tested by using male mice of the ddY line and male rats of the Wistar line, it was found that no animal died even if the anti-retroviral agent of the present invention obtained in the above-mentioned Specific Example was orally administered at a dose of 15 g/kg (administration limit).

As seen from this result, the anti-retroviral agent of the present invention comprising Xiao-Chai-Hu-Tang has a very low toxicity and a high safety. Note, Xiao-Chai-Hu-Tang has been clinically used as a Chinese herb medicine preparation for many decades, and it has been confirmed that this preparation has substantially no adverse side effects.

When the acute toxicity test of baicalin, baicalein, quercetin, and hesperetin was carried out by using male mice of the ddY line and male rats of the Wistar line, it was found that no animal died by oral administration at a dose of 1 g/kg. As apparent from this result, baicalin, baicalein, quercetin, and hesperetin have a very low toxicity and a high safety.

It is known that the $LD_{50}$ values of alizarin and Alizarine Red S upon intravenous administration are 120 mg/kg and 70 mg/kg, respectively, and accordingly, it is obvious that the compound of formula I also has a very low toxicity and a high safety.

In view of the experimental data of the present invention and the acute toxicity test results, it is considered that the effective administration amount of the anti-retroviral agent comprising Xiao-Chai-Hu-Tang is such that 1 to 10 g of the agent as the dry extract powder can be divided into three doses and administered three times per day for an adult, though the effective administration amount differs to some extent according to the age and body weight of the patient and the seriousness of the disease.

Administration amounts and formulation methods for baicalin, baicalein, quercetin, and hesperetin will now be described.

Baicalin, baicalein, quercetin, hesperetin, and the compound of formula I can be administered to animals and humans directly or together with customarily used pharmaceutical carriers. The administration form is not particularly critical and an appropriate administration form is selected according to need. For example, there can be mentioned oral medicines such as tablets, capsules, granules, fine granules and powders, and non-oral medicines such as injections and suppositories.

The dose of the oral medicine required to obtain the intended effect differs to some extent according to the age and body weight of the patient and the degree of the disease, but preferably 100 to 6000 mg as the weight of baicalin, baicalein, quercetin, hesperetin or the compound of formula I is generally administered dividedly several times per day for an adult.

In the present invention, oral medicines such a tablets, capsules and granules are prepared by customary procedures using, for example, starch, lactose, refined sugar, mannitol, carboxymethyl cellulose, corn starch, and inorganic salts.

For formation of these medicines, binders, disintegrating agents, surface active agents, lubricants, flowability improvers, taste improvers, colorants, and perfumes can be used in addition to the above-mentioned excipients. Specific examples thereof are described below.

(Binders)

Starch, dextrin, gum arabic, gelatin, hydroxypropylstarch, methylcellulose, sodium carboxymethyl cellulose, hydroxypropyl cellulose, crystalline cellulose, ethylcellulose, polyvinyl pyrrolidone, and macrogall.

(Disintegrating Agents)

Starch, hydroxypropylstarch, sodium carboxymethyl cellulose, calcium carboxymethyl cellulose, carboxymethyl cellulose, and lower substituted hydroxypropyl cellulose.

(Surface Active Agents)

Sodium lauryl sulfate, soybean lecithin, sucrose, fatty acid ester, aluminum stearate, and Polysolvate 80.

(Lubricants)

Talc, wax, hydrogenated vegetable oil, sucrose fatty acid ester, magnesium stearate, calcium stearate, aluminum stearate, and polyethylene glycol.

(Flowability Improvers)

Light silicic anhydride, dry aluminum hydroxide gel, synthetic aluminum silicate, and magnesium silicate.

Baicalin, baicalein, quercetin, hesperetin, and the compound of formula I can be administered in the form of suspensions, emulsions, syrups, and elixirs. These preparations may further contain a taste improver, a smell improver, and a colorant.

The dosage of the non-oral medicine for obtaining the intended effect varies to some extent depending on the age and body weight of the patient and the degree of the disease, but preferably is administered as 1 to 100 mg as the weight of baicalein, baicalin, quercetin, hesperetin or the compound of formula I per day for an adult by intravenous injection, intravenous drip, hypodermic injection, or intramuscular injection.

This non-oral medicine is prepared by customary procedures. As the diluent, there can be used distilled water for injection, physiological saline solution, an aqueous solution of glucose, a vegetable oil for injection, sesame oil, coconut oil, soybean oil, corn oil, propylene glycol and polyethylene glycol. A fungicide, an antiseptic agent and a stabilizer can be added according to need. In view of the stability of the non-oral medicine, a method can be adopted in which the non-oral medicine is filled in a vial and frozen, water is removed by the conventional freeze-drying technique, and the liquid is prepared again just before application. An isotropic agent, a stabilizer, an antiseptic agent, and an analgesic agent can be added according to need.

As other non-oral agents, there can be mentioned coating agents such as external lotions and ointments, and suppositories for intrarectal administration. These non-oral medicines can be prepared by customary procedures.

The anti-retroviral agent of the present invention will now be described in detail with reference to the following examples, that by no means limit the scope of the invention.

Example 1

The dry extract powder (200 g) obtained in the Specific Example was mixed with 89 g of lactose and 1 g of magnesium stearate and the mixture was tableted by a single-shot tableting machine to form slug tablets having a diameter of 20 mm and a weight of about 2.3 g. The tablets were pulverized by an oscillator, subjected to particle size adjustment, and sieved to obtain a good granule consisting of particles having a size of 20 to 50 mesh.

This granule was administered at a dose of 0.5 to 4.5 g (corresponding to 0.34 to 3.10 g of the dry extract powder) three times a day according to the disease conditions.

Example 2

The dry extract powder (200 g) obtained in the Specific Example was mixed with 20 g of microcrystalline cellulose and 5 g of magnesium stearate, and the mixture was tableted by a one-shot tableting machine to form tablets having a diameter of 7 mm and a weight of 225 mg. Each tablet contained 200 mg of the dry extract powder of the anti-retroviral agent of the present invention. Two to sixteen tablets were administered three times a day according to the disease conditions.

Example 3

The dry extract powder obtained in the Specific Example was filled in hard capsules so that each capsule contained 500 mg of the powder. Two to twenty capsules were administered three times a day according to the disease conditions.

Example 4

| | | |
|---|---|---|
| (1) | Corn starch | 44 g |
| (2) | Crystalline cellulose | 40 g |
| (3) | Calcium carboxymethyl cellulose | 5 g |
| (4) | Light silicic anhydride | 0.5 g |
| (5) | Magnesium stearate | 0.5 g |
| (6) | Baicalin | 10 g |
| | Total | 100 g |

According to the above-mentioned recipe, the components (1) through (6) were homogeneously mixed, and the mixture was compression-molded by a tableting machine to obtain tablets, each having a weight of 200 mg.

Each tablet contained 20 mg of baicalin, and 5 to 7 tablets were dividedly administered several times per day for an adult.

Example 5

| | | |
|---|---|---|
| (1) | Crystalline cellulose | 84.5 g |
| (2) | Magnesium stearate | 0.5 g |
| (3) | Calcium carboxymethyl cellulose | 5 g |
| (4) | Baicalein | 10 g |
| | Total | 100 g |

According to the above-mentioned recipe, the components (1) and (4) and a part of the component (2) were homogeneously mixed, and the mixture was compression-molded and then pulverized and the component (3) and the remainder of the component (2) were added to the pulverization product. The mixture was compression-molded by a tableting machine to form tablets, each having a weight of 200 mg.

Each tablet contained 20 mg of baicalein, and 5 to 7 tablets were dividedly administered several times per day for an adult.

Example 6

| | | |
|---|---|---|
| (1) | Crystalline cellulose | 34.5 g |
| (2) | 10% Ethanol solution of hydroxypropyl cellulose | 50 g |
| (3) | Calcium carboxymethyl cellulose | 5 g |
| (4) | Magnesium stearate | 0.5 g |
| (5) | Baicalin | 10 g |
| | Total | 100 g |

According to the above-mentioned recipe, the components (1), (2) and (5) were homogeneously mixed, and according to customary procedures, the mixture was kneaded, granulated by an extrusion granulator, dried, and disintegrated. Then the disintegration product was mixed with the components (3) and (4) and the mixture was compression-molded by a tableting machine to obtain tablets each having a weight of 200 mg.

Each tablet contained 20 mg of baicalin, and 5 to 7 tablets were dividedly administered several times per day for an adult.

Example 7

| | | |
|---|---|---|
| (1) | Corn starch | 84 g |
| (2) | Magnesium stearate | 0.5 g |
| (3) | Calcium carboxymethyl cellulose | 5 g |
| (4) | Light silicic anhydride | 0.5 g |
| (5) | Baicalein | 10 g |
| | Total | 100 g |

According to the above-mentioned recipe, the components (1) through (5) were homogeneously mixed, and the mixture was compression-molded by a compression molding machine, pulverized by a pulverizer and sieved to obtain a granule.

One gram of this granule contained 100 mg of baicalein, and 1 to 2.5 g of the granule was dividedly administered several times per day for an adult.

Example 8

| | | |
|---|---|---|
| (1) | Crystalline cellulose | 40 g |
| (2) | 10% Ethanol solution of hydroxypropyl cellulose | 50 g |
| (3) | Baicalin | 10 g |
| | Total | 100 g |

According to the above-mentioned recipe, the components (1) through (3) were homogeneously mixed and kneaded. The kneaded mixture was granulated by a granulator, dried, and sieved to obtain a granule.

One gram of this granule contained 100 mg of baicalin, and 1 to 2.5 g of the granule was dividedly administered several times per day for an adult.

Example 9

| | | |
|---|---|---|
| (1) | Corn starch | 89.5 g |
| (2) | Light silicic anhydride | 0.5 g |
| (3) | Baicalein | 10 g |
| | Total | 100 g |

According to the above-mentioned recipe, the components (1) through (3) were homogeneously mixed, and the mixture was filled in capsules No. 2 so that each capsule contained 200 mg of the mixture.

Each capsule contained 20 mg of baicalein, and 5 to 7 capsules were dividedly administered several times per day for an adult.

Example 10

| | | |
|---|---|---|
| (1) | Distilled water for injection | appropriate amount |
| (2) | Glucose | 200 mg |
| (3) | Baicalin | 10 mg |
| | Total | 15 ml |

The components (2) and (3) were dissolved in distilled water for injection, and the solution was filled in an ampoule having a capacity of 5 ml. Sterilization was carried out at 121 ° C for heating under pressure to obtain an injection.

Example 11

| | | |
|---|---|---|
| (1) | Crystalline cellulose | 84.5 g |
| (2) | Magnesium stearate | 0.5 g |
| (3) | Calcium carboxymethyl cellulose | 5 g |
| (4) | Quercetin | 10 g |
| | Total | 100 g |

According to the above-mentioned recipe, the components (1) and (4) and a part of the component (2) were homogeneously mixed and the mixture was compression-molded and pulverized. Then the component (3) and the remainder of the component (2) were added to the pulverization product, and the mixture was compression-molded by a tableting machine to obtain tablets, each having a weight of 200 mg.

Each tablet contained 20 mg of quercetin, and 5 to 7 tablets were dividedly administered several times per day for an adult.

Example 12

| | | |
|---|---|---|
| (1) | Corn starch | 84 g |
| (2) | Magnesium stearate | 0.5 g |
| (3) | Calcium carboxymethyl cellulose | 5 g |
| (4) | Light silicic anhydride | 0.5 g |
| (5) | Quercetin | 10 g |
| | Total | 100 g |

According to the above-mentioned recipe, the components (1) through (5) were homogeneously mixed and the mixture was compression-molded by a compression molding machine, pulverized by a pulverizer and sieved to obtain a granule.

One gram of this granule contained 100 mg of quercetin, and 1 to 2.5 g of the granule was dividedly administered several times per day for an adult.

Example 13

| | | |
|---|---|---|
| (1) | Corn starch | 89.5 g |
| (2) | Light silicic anhydride | 0.5 g |
| (3) | Quercetin | 10 g |
| | Total | 100 g |

According to the above-mentioned recipe, the components (1) through (3) were homogeneously mixed and the mixture was filled in capsules No. 2 so that each capsule contained 200 mg of the mixture.

Each capsule contained 20 mg of quercetin, and 5 to 7 capsules were dividedly administered several timer per day for an adult.

Example 14

| | | |
|---|---|---|
| (1) | Distilled water for injection | appropriate amount |
| (2) | Glucose | 200 mg |
| (3) | Quercetin | 10 mg |
| | Total | 15 ml |

The components (2) and (3) were dissolved in distilled water for injection and the solution was filled in an ampoule having a capacity of 5 ml and subjected to sterilization at 121 ° C for 15 minutes under pressure to obtain an injection.

Example 15

| | | |
|---|---|---|
| (1) | Crystalline cellulose | 84.5 g |
| (2) | Magnesium stearate | 0.5 g |
| (3) | Calcium carboxymethyl cellulose | 5 g |
| (4) | Hesperetin | 10 g |
| | Total | 100 g |

According to the above-mentioned recipe, the components (1) and (4) and a part of the component (2) were homogeneously mixed and the mixture was compression-molded and pulverized. Then the component (3) and the remainder of the component (2) were added to the pulverization product, and the mixture was compression-molded by a tableting machine to obtain tablets, each having a weight of 200 mg.

Each tablet contained 20 mg of hesperetin, and 5 to 7 tablets were dividedly administered several times per day for an adult.

Example 16

| | | |
|---|---|---|
| (1) | Corn starch | 84 g |
| (2) | Magnesium stearate | 0.5 g |
| (3) | Calcium carboxymethyl cellulose | 5 g |
| (4) | Light silicic anhydride | 0.5 g |
| (5) | Hesperetin | 10 g |
| | Total | 100 g |

According to the above-mentioned recipe, the components (1) through (5) were homogeneously mixed and the mixture was compression-molded by a compression molding machine, pulverized by a pulverizer and sieved to obtain a granule.

One gram of this granule contained 100 mg of hesperetin, and 1 to 2.5 g of the granule was dividedly administered several times per day for an adult.

Example 17

| | | |
|---|---|---|
| (1) | Corn starch | 89.5 g |
| (2) | Light silicic anhydride | 0.5 g |
| (3) | Hesperetin | 10 g |
| | Total | 100 g |

According to the above-mentioned recipe, the components (1) through (3) were homogeneously mixed and the mixture was filled in capsules No. 2 so that each capsule contained 200 mg of the mixture.

Each capsule contained 20 mg of hesperetin, and 5 to 7 capsules were dividedly administered several times per day for an adult.

Example 18

| | | |
|---|---|---|
| (1) | Distilled water for injection | appropriate amount |
| (2) | Glucose | 200 mg |
| (3) | Hesperetin | 10 mg |
| | Total | 15 ml |

The components (2) and (3) were dissolved in distilled water for injection, and the solution was filled in an ampoule having a capacity of 5 ml and subjected to sterilization at 121°C for 15 minutes under pressure to obtain an injection.

Example 19

| | | |
|---|---|---|
| (1) | Corn starch | 44 g |
| (2) | Crystalline cellulose | 40 g |
| (3) | Calcium carboxymethyl cellulose | 5 g |
| (4) | Light silicic anhydride | 0.5 g |
| (5) | Magnesium stearate | 0.5 g |
| (6) | Alizarin | 10 g |
| | Total | 100 g |

According to the above-mentioned recipe, the components (1) through (6) were homogeneously mixed, and the mixture was compression-molded by a tableting machine to obtain tablets, each having a weight of 200 mg.

Each tablet contained 20 mg of alizarin, and 5 to 7 tablets were dividedly administered several times per day for an adult.

Example 20

| | | |
|---|---|---|
| (1) | Crystalline cellulose | 84.5 g |
| (2) | Magnesium stearate | 0.5 g |
| (3) | Calcium carboxymethyl cellulose | 5 g |
| (4) | Alizarin complexon | 10 g |
| | Total | 100 g |

According to the above-mentioned recipe, the components (1) and (4) and a part of the component (2) were homogeneously mixed, and the mixture was compression-molded and pulverized. Then the component (3) and the remainder of the component (2) were added to the pulverization product, and the mixture was compression-molded by a tableting machine to obtain tablets, each having a weight of 200 mg.

Each tablet contained 20 mg of alizarin complexon, and 5 to 7 tablets were dividedly administered several times per day for an adult.

Example 21

| | | |
|---|---|---|
| (1) | Crystalline cellulose | 34.5 g |
| (2) | 10% Ethanol solution of hydroxypropyl cellulose | 50 g |
| (3) | Calcium carboxymethyl cellulose | 5 g |
| (4) | Magnesium stearate | 0.5 g |
| (5) | Alizarine Red S | 10 g |
| | Total | 100 g |

According to the above-mentioned recipe, the components (1), (2) and (5) were homogeneously mixed, and according to customary procedures, the mixture was kneaded, granulated by an extrusion granulator, dried and disintegrated. Then the components (3) and (4) were added to the disintegration product and the mixture was compression-molded by a tableting machine to obtain tablets each having a weight of 200 mg.

Each tablet contained 20 mg of Alizarine Red S, and 5 to 7 tablets were dividedly administered several times per day for an adult.

Example 22

| | | |
|---|---|---|
| (1) | Corn starch | 84 g |
| (2) | Magnesium stearate | 0.5 g |
| (3) | Calcium carboxymethyl cellulose | 5 g |
| (4) | Light silicic anhydride | 0.5 g |
| (5) | Quinalizarin | 10 g |
| | Total | 100 g |

According to the above-mentioned recipe, the components (1) through (5) were homogeneously mixed, and the mixture was compression-molded by a compression molding machine, pulverized by a pulverizer, and sieved to obtain a granule.

One gram of this granule contained 100 mg of quinalizarin, and 1 to 2.5 g of the granule was dividedly administered several times per day for an adult.

Example 23

| | | |
|---|---|---|
| (1) | Crystalline cellulose | 55 g |
| (2) | 10% Ethanol solution of hydroxypropyl cellulose | 35 g |
| (3) | Purpurin | 10 g |
| | Total | 100 g |

According to the above-mentioned recipe, the components (1) through (3) were homogeneously mixed, and the mixture was kneaded, granulated by an extrusion granulator, dried, and sieved to obtain a granule.

One gram of this granule contained 100 mg of purpurin, and 1 to 2.5 g of the granule was dividedly administered several times per day for an adult.

Example 24

| | | |
|---|---|---|
| (1) | Corn starch | 89.5 g |
| (2) | Light silicic anhydride | 0.5 g |
| (3) | Alizarin complexon | 10 g |
| | Total | 100 g |

According to the above-mentioned recipe, the components (1) through (3) were homogeneously mixed, and the mixture was filled in capsules No. 2 so that each capsule contained 200 mg of the mixture.

Each capsule contained 20 mg of alizarin complexon, and 5 to 7 capsules were dividedly administered several times per day for an adult.

Example 25

| | | |
|---|---|---|
| (1) | Distilled water for injection | appropriate amount |
| (2) | Glucose | 100 mg |
| (3) | Alizarin complexon | 10 mg |
| | Total | 15 ml |

The components (2) and (3) were dissolved in distilled water for injection, and the solution was filled in an ampoule having a capacity of 5 ml and subjected to sterilization at 121 °C for 15 minutes under pressure to obtain an injection.

**Claims**

1. Use of at least one substance selected from Xiao-Chai-Hu-Tang, baicalein, baicalin, quercetin, hesperetin, and alizarin and derivatives and pharmacologically acceptable salts thereof represented by the following formula I:

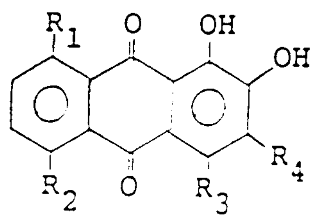

I

wherein $R_1$, $R_2$ and $R_3$, which may be the same or different, stand for a hydrogen atom or a hydroxyl group, and $R_4$ stands for a hydrogen atom, a sulfonic acid group or a dicarboxymethylaminomethyl group, in the manufacture of an anti-retroviral agent.

2. Use according to claim 1 wherein the anti-retroviral agent is manufactured using Xiao-Chai-Hu-Tang which is prepared from 4 to 7 parts by weight of Bupleuri radix, 3 parts by weight of Scutellariae radix, 2 parts by weight of Glycyrrhizae radix, 2 to 3 parts by weight of Ginseng radix, 1 part by weight of Zingiberis rhizoma, 2 to 3 parts by weight of Zizyphi fructus, and 4 to 5 parts by weight of Pinelliae tuber.

3. Use according to claim 2 wherein the Xiao-Chai-Hu-Tang is obtained in the form of a dry extract powder, and 4.5 g of the dry extract powder contains 25.0 to 52.0 mg of glycyrrhizin, 90 to 210 mg of baicalin and 2.3 to 6.9 mg of saikosaponin b2.

4. Use according to claim 2 wherein the anti-retroviral agent is for administration to a human adult patient in an amount of from 1 to 10 g per day.

5. Use as set forth in claim 1, wherein the effective ingredient is selected from baicalein, baicalin, quercetin, hesperetin, and compounds of the formula I and salts thereof, and the anti-retroviral agent is administered in an amount of 100 to 6000 mg as the effective ingredient per day for an adult.

6. Use according to claim 5 wherein the compound of the formula I is selected from alizarin, alizarin complexone, Alizarine Red S, quinalizarin and purpurin.

## Patentansprüche

1. Verwendung von mindestens einer Substanz, die aus Xiao-Chai-Hu-Tang, Baicalein, Baicalin, Qeurcetin, Hesperetin und Alizarin sowie Derivaten und pharmakologisch geeigneten Salzen davon ausgewählt ist und durch die nachfolgende Formel I

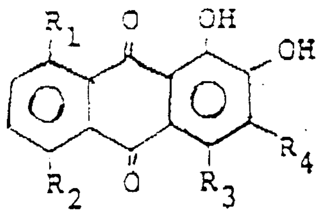

I

repräsentiert wird, worin $R_1$, $R_2$ und $R_3$, die gleich oder verschieden sein können, ein Wasserstoffatom oder eine Hydroxylgruppe und $R_4$ ein Wasserstoffatom, eine Sulfonsäuregruppe oder eine Dicarboxymethylaminomethylgruppe bedeuten, bei der Herstellung eines anti-retroviralen Arzneimittels.

2. Verwendung nach Anspruch 1, bei der das anti-retrovirale Arzneimittel unter Verwendung von Xiao-Chai-Hu-Tang hergestellt wird, das aus 4 bis 7 Gewichtsteilen Bupleuri radix, 3 Gewichtsteilen Scutellariae radix, 2 Gewichtsteilen Glycyrrhizae radix, 2 bis 3 Gewichtsteilen Ginseng radix, 1 Gewichtsteil Zingiberis rhizoma, 2 bis 3 Gewichtsteilen Zizyphi fructus und 4 bis 5 Gewichtsteilen Pinelliae tuber gewonnen wird.

3. Verwendung nach Anspruch 2, bei der das Xiao-Chai-Hu-Tang in der Form eines Trockenpulverextraktes erhalten wird und 4,5 g des Trockenpulverextraktes 25,0 bis 52,0 mg Glycyrrhizon, 90 bis 210 mg Baicalin und 2,3 bis 6,9 mg Saikosaponin b2 enthält.

4. Verwendung nach Anspruch 2, bei der das anti-retrovirale Arzneimittel für eine Darreichung an einen menschlichen erwachsenen Patienten in einer Menge von 1 bis 10 g pro Tag bestimmt ist.

5. Verwendung nach Anspruch 1, bei der der Wirkstoff aus Baaicalein, Baicalin, Quercetin, Hesperetin und Verbindungen der Formel I und Salzen derselben ausgewählt ist und das anti-retrovirale Arzneimittel in einer Menge von 100 bis 6000 mg als Wirkstoff pro Tag an einen Erwachsenen verabreicht wird.

6. Verwendung nach Anspruch 5, bei der die Verbindung der Formel I aus Alizarin, Alizarin complexon, Alizarin Red S, Chinalizarin und Purpurin ausgewählt ist.

## Revendications

1. Utilisation d'au moins une substance choisie parmi Xiao-Chai-Hu-Tang, baicaléine, baicaline, quercétine, hespérétine et alizarine, et leurs dérivés, et leurs sels pharmacologiquement acceptables, représentée par la formule I:

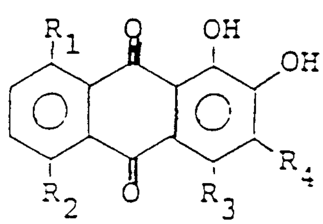

I

dans laquelle:

| | |
|---|---|
| $R_1$, $R_2$ et $R_3$ | qui peuvent être identiques ou différents, représentent un atome d'hydrogène ou un groupe hydroxyle; et |
| $R_4$ | représente un atome d'hydrogène, un groupe acide sulfonique ou un groupe |

dicarboxyméthylaminométhyle,
dans la préparation d'un agent anti-rétroviral.

2. Utilisation selon la revendication 1, caractérisée en ce que l'on prépare l'agent anti-rétroviral en utilisant Xiao-Chai-Hu-Tang qui est lui même préparé à partir de 4 à 7 parties en poids de Bupleuri radix, 3 parties en poids de Scutellariae radix, 2 parties en poids de Glycyrrhizae radix, 2 à 3 parties en poids de Ginseng radix, une partie en poids de Zingiberis rhizoma, 2 à 3 parties en poids de Zizyphi fructus et de 4 à 5 parties en poids de Pinalliae tuber.

3. Utilisation selon la revendication 2, caractérisée en ce que le Xiao-Chai-Hu-Tang est obtenu sous la forme d'une poudre d'extrait sec, et en ce que 4,5 g de la poudre sec contiennent de 25,0 à 52,0 mg de glycyrrhizine, de 90 à 210 mg de baicaline et de 2,3 à 6,9 mg de saikosaponine b2.

4. Utilisation selon la revendication 2, caractérisée en ce que l'agent anti-rétroviral est destiné à l'administration à un patient adulte humain en quantité de 1 à 10 g/j.

5. Utilisation selon la revendication 1, caractérisée en ce que l'ingrédient efficace est choisi parmi baicaléine, baicaline, quercétine, hespérétine et leurs dérivés de formule I et leurs sels, et en ce que l'agent anti-rétroviral est administré en quantité de 100 à 6 000 mg d'ingrédient efficace par jour pour un adulte.

6. Utilisation selon la revendication 5, caractérisée en ce que le dérivé de formule est choisi parmi alizarine, alizarine complexone, rouge S d'alizarine, quinalizarine et purpurine.